# EUROPEAN PATENT APPLICATION

(11) **EP 4 559 890 A1**
(43) Date of publication of application: **28.05.2025**
(21) Application number: 23212216.8
(22) Date of filing: 27.11.2023
(51) Int. Cl.: C07B 59/00, A61K 51/00

(54) **STABILIZED METHOD FOR 18F-LABELING**

(71) Applicant: Trasis S.A., 4430 Ans (BE)
(72) Inventor: PHILIPPART, Gauthier, 4280 AVIN (BE); MORELLE, Jean-Luc, 4000 LIEGE (BE); WARNIER, Corentin, 4870 NESSONVAUX (BE); TRUMP, Laura, 4000 LIEGE (BE); GERARDY, Romaric, 4030 LIEGE (BE); CERFONTAINE, Simon, 1150 WOLUWE-SAINT-PIERE (BE)
(74) Representative: AWA Benelux

(57) **Abstract**

The present invention relates to a method for stabilizing the production yield of [18F]-labelled radiotracers by the addition of a radical scavenger in the reaction media of the nucleophilic [18F]-fluorination step.

## Description

### Field of the invention

The invention pertains to the field of chemical synthesis. More particularly, the invention pertains to methods for the synthesis of [¹⁸F]-labelled molecules, and more particularly to a method for stabilizing the radiolabelling outcome at high activity.

### Background and Prior Art

### Positron Emission tomography (PET) and [¹⁸F]-labeling

Positron Emission Tomography (PET) imaging is a minimally invasive useful imaging technique that can produce three-dimensional images of processes in the body. PET measures the physiological function by observing blood flow, metabolism, neurotransmitters and radiolabelled agents. This imaging technique is based on the indirect detection of gamma rays and radiation emitted by a radioactive agent that is injected into the body.

Of all the positron emitters available for PET, fluorine-18 (¹⁸F) is the most ideal due to its favorable decay scheme. The half-life of this radionuclide (110 min) allows time for multistep radiolabelling reaction and for delivery of ¹⁸F-labelled tracers to distant user sites. Moreover, the short positron range (2.3 mm) in tissue and the ideal decay process (97% positron emission) contribute to offer high resolution images. As a result, the vast majority of PET tracers used in the clinic are ¹⁸F-labelled molecules, such as the [¹⁸F] isotope-labeled glucose, [¹⁸F]2-Fluoro-2-Deoxy-D-glucose (hereinafter [¹⁸F]-FDG), that has become world widely used in nuclear medicine for diagnostic studies using a PET body scanning technique.

[¹⁸F]-fluoride is produced by irradiation of 'enriched' water, containing H₂¹⁸O, with protons resulting in the reaction ¹⁸O(p,n)¹⁸F. Only a minor fraction of the [¹⁸O] is converted. The [¹⁸F] isotope is then separated from the water and processed for production of a radiopharmaceutical agent.

In the current practice, fluoride recovery is based on the use of an anion-exchange resin. The recovery is carried out in two steps, extraction and elution: first the anions (not only fluoride) are separated from the enriched [¹⁸O] water and trapped on the said resin. The anions, including [¹⁸F]-fluoride, are then eluted into a mixture containing water, organic solvents, a phase transfer agent or activating agent or phase transfer catalyst, such as for example the complex potassium carbonate-Kryptofix 222 (K₂CO₃-K₂₂₂) or a tetrabutyl-ammonium salt. The [¹⁸F] fluoride radiochemical recovery yield is very effective, usually exceeding 99%.

The most usual labeling method, known as *nucleophilic substitution*, requires anhydrous or very low water content solutions. Thus, an evaporation step (or drying step) is usually necessary after recovery to remove the excess water. It usually consists in multiple azeotropic evaporations of acetonitrile or of low boiling temperature organic solvent. Such evaporations require several minutes and these steps result in the creation of "naked", highly nucleophilic [¹⁸F]-fluoride.

The subsequent [¹⁸F]-fluorination step can be classified as a nucleophilic aliphatic or aromatic substitution. A prerequisite for [¹⁸F]-substitutions is the presence of a good leaving group on the precursor to be labelled. Along these lines, the halides Cl, Br and I or the different types of sulfonates - tosylates, nosylate, mesylate and triflate - are used most often in radiofluorination reactions. After evaporation, the precursor dissolved in a polar aprotic solvent is added to the naked [¹⁸F]-fluoride anions in the reactor and the nucleophilic substitution occurs. Usually, the radiolabeling requires the reactor to be heated. Alternatively, the nucleophilic substitution can be conducted on a solid-support cartridge, by loading the precursor solution on a cartridge which previously trapped the [¹⁸F]-fluoride anions. The cartridge may be heated by a heating device to enhance the nucleophilic substitution reaction. In any case, the radiolabeling step, e.g. the nucleophilic substitution, output is crucial for the radiotracer synthesis outcome.

Generally, nucleophilic fluorination reaction is carried out in a polar aprotic solvent, such as acetonitrile (ACN), N,N-dimethylformamide (DMF), or dimethyl sulfoxide (DMSO), to increase the solubility of fluorine salt and the reactivity of fluoride. It is known that protic solvents, such as water or alcohol, are not suitable for the nucleophilic fluorination reaction since protic solvents reduce the nucleophilicity of [¹⁸F]-fluoride by extensive hydrogen bonding and interaction with the partial positive charge of these solvents. Therefore, polar aprotic solvents, such as the aforementioned ACN, DMF and DMSO have been widely used as a solvent for these [¹⁸F]-radiolabeling reactions. The nucleophilicity of [¹⁸F]fluoride in polar aprotic solvents usually is enhanced through the selective solvation of its counter cations by the negative end of the polar aprotic solvent dipole and the lack of a proton for hydrogen bonding, which leave the [¹⁸F]-fluoride free or naked. However, in an effort to optimize labeling yields, a highly efficient aliphatic nucleophilic fluorination and [¹⁸F]-radiofluorination method with alkali metal fluorides or [¹⁸F]-fluoride using nonpolar protic tert-alcohols as a reaction solvent has been reported (Kim D.W., Ahn D.S., Oh Y.H., Lee S., Kil H.S., Oh S.J. A new class of SN2 reactions catalyzed by protic solvents: facile fluorination for isotopic labeling of diagnostic molecules. J Am Chem Soc (2006) 128:16393-16397; Patent application WO2006065038A1). In this method, the tert-alcohol media, such as tert-butyl alcohol and tert-amyl alcohol, greatly enhanced the reactivity of alkali metal fluorides. In particular, the use of tert-alcohols could reduce the formation of typical byproducts (namely, alkenes, alcohols, or ethers) dramatically in the nucleophilic fluorination of base-sensitive compounds, such as 1-(2-mesylethyl)naphthalene and N-5-bromopentanoyl-3,4-dimethoxyaniline compared with the use of conventional polar aprotic solvents (Kim D.W., Ahn D.S., Oh Y.H., Lee S., Kil H.S., Oh S.J. A new class of SN2 reactions catalyzed by protic solvents: facile fluorination for isotopic labeling of diagnostic molecules. J Am Chem Soc (2006) 128:16393-16397; Kim D.W., Jeong H.J., Lim S.T., Sohn M.H., Katzenellenbogen J.A., Chi D.Y., Facile nucleophilic fluorination reaction using tert-alcohols as a reaction medium: significantly enhanced reactivity of alkali metal fluorides and improved selectivity. J Org Chem (2008) 73:957-962; Kim D.W., Jeong H.J., Lim S.T., Sohn M.H., Chi D.Y., Facile nucleophilic fluorination by synergistic effect between polymersupported ionic liquid catalyst and tert-alcohol. Tetrahedron (2008) 64:4209-4214). On the contrary, results show that the use of primary or secondary alcohols, which are polar protic solvents, such as ethanol, lead to the production of by-products because of their polar, thus strongly lowering the labeling yield.

Because this finding was in marked contrast with the expectations from the conventional [¹⁸F]-radiofluorination pathway; the mechanism of the nucleophilic fluorination reaction in nonpolar protic tert-alcohol solvent has been reported (Kim D.W., Jeong H.J., Lim S.T., Sohn M.H., Recent Trends in the Nucleophilic [18Fj-radio/abeling Method with No-carrier-added [18F]fluoride. Nucl Med Mol Imaging (2010) 44:25-32). The authors suggested the mechanism: limited solvation of [¹⁸F]-fluoride coordinated with bulky tert alcohols (named "flexible" fluoride) could make the fluoride an especially good nucleophile in this medium; leaving group reactivity could be enhanced by hydrogen bonding between the tert-alcohol solvent with oxygen atoms of a sulfonate leaving group; side reactions, such as eliminations, hydroxylations, and intramolecular alkylations, could be suppressed by the protic environment, particularly when using base-sensitive precursors (Kim D.W., Jeong H.J., Lim S.T., Sohn M.H., Katzenellenbogen J.A., Chi D.Y., Facile nucleophilic fluorination reaction using tert-alcohols as a reaction medium: significantly enhanced reactivity of alkali metal fluorides and improved selectivity. J Org Chem (2008) 73:957-962; Kim D.W., Jeong H.J., Lim S.T., Sohn M.H., Chi D.Y., Facile nucleophilic fluorination by synergistic effect between polymersupported ionic liquid catalyst and tert-alcohol. Tetrahedron (2008) 64:4209-4214). In a more recent paper, it was proven that "flexible" fluoride could be formed in the tert-alcohol media fluorination processing as a result of the preparation of tetrabutylammonium tetra t-butanol coordinated fluoride, TBAF(t-BuOH)₄, from commercially available TBAF in t-BuOH. This TBAF(t-BuOH)₄ also showed good performance in the nucleophilic fluorination because of its ideal favorable properties as a fluoride source, such as the dehydrated state for anhydrous reaction conditions, low hygroscopicity, good solubility in organic solvents, and good nucleophilicity with low basicity (Kim D.W., Jeong H.J., Lim S.T., Sohn M.H. Tetrabutylammonium tetra(tert-butyl alcohol)-coordinated fluoride as a facile fluoride source. Angew Chem Int Ed Engl (2008) 47:8404-8406). To achieve such a 'flexible' fluoride, the tert-alcohol must be present predominantly in the reaction media, e.g. as the solvent itself and not added as an adjuvant or a catalyst. Obviously, such a 'flexible' fluoride is not accessible using polar protic solvent such as primary or secondary alcohol, mainly because of their high intrinsic polarity.

Protecting groups are often necessary to eliminate acidic protons in the molecule that would diminish the nucleophilicity of [¹⁸F]-fluoride. If protecting groups have been used, they must be removed after the radiolabeling step. Finally, the product has to be purified by Solid Phase Extraction (SPE) or preparative High-Performance Liquid Chromatography (HPLC) and formulated for injection.

### Radiolysis

Because of the aforementioned short half-life of the [¹⁸F] isotope, [¹⁸F]-labelled radiotracers must be produced in relatively large activities to allow for decay during delivery to the patient from a manufacturing facility. It is well known that the major stability issue for radiopharmaceuticals is radiolysis which can be either auto-radiolysis, self-destruction by its own radiation, and/or attack by free radicals formed by the radiation on environmental species. The radiolysis, or more specifically auto-radiolysis, of [¹⁸F]-FDG has been extensively reviewed and mainly results in free [¹⁸F]-fluoride ions (Jószai I., Svidró M., Pótári N., Recommendations for selection of additives for stabilization of [18F]FDG. Appl Radiat Isot. (2019) 146:78-83) and free radicals because of the reaction of ionizing radiation with water (Buriova E., Macasek F., Kropacek M., Prochazka L., Autoradiolysis of 2-deoxy-2-[18F]fluoro-D-glucoseradiopharmaceutical. J Radioanal Nucl Chem. (2005) 3:595-602).

There are several strategies to reduce radiolysis: one common and well documented strategy is to stabilize the final product with radio-stabilizers. The most well-known and frequently used radio-stabilizer for [¹⁸F]-FDG is ethanol. Adding ethanol to the formulation of the final product in 0,1 - 0,4 % concentration has proven effective to stabilize [¹⁸F]-FDG with activity up to 25 GBq/ml up to 16 hours (Dantas N.M., Nascimento J.E., Santos-Magalhães N.S., Oliveira M.L., Radiolysis of2-[18F]fluoro-2 deoxy-D-glucose ([18F]FDG) and the role of ethanol, radioactive concentration and temperature of storage. Appl Radiat Isot. (2013) 72:158-62; Fawdry R.M., Radiolysis of 2-[18F]fluoro-2-deoxy-D-glucose (FDG) and the role of reductant stabilisers. Appl Radiat Isot. (2007) 65:1193-201; Jacobson M.S., Dankwart H.R., Mahoney D.W., Radiolysis of 2-[18F]fluoro-2-deoxy-D-glucose([18F]FDG) and the role of ethanol and radioactive concentration. Appl Radiat Isot. (2009) 67:990-5; Walters L.R., Martin K.J., Jacobson M.S., Hung J.C., Mosman E.A., Stability evaluation of 18F-FDG at high radioactive concentrations. J Nucl Med. (2011) 52(supplement 1):2413; Patent application US7018614B2 ; Mosdzianowski C., Lemaire C., Simoens F., Aerts J., Morelle J.-L., Luxen A., Epimerization study on [18F]FDG produced by an alkaline hydrolysis on solid support under stringent conditions. Appl Radiat Isot. (2002) 56:871-5; Jószai I., Svidró M., Pótári N., Recommendations for selection of additives for stabilization of [18F]FDG. Appl Radiat Isot. (2019) 146:78-83; Holler J.G., Renmaelmo B., Fjellaksel R., Stability evaluation of [18F]FDG: literature study, stability studies from two different PET centres and future recommendations. EJNMMI Radiopharmacy and Chemistry (2022) 7:2-20)

The dilution factor for the product as a stabilizer must also be considered, but the dilution has limitations due to the maximum volume to be injected, and additionally a radio-stabilizer can be considered (Jiménez Romero I.R., Roca Engronyat M., Campos Añón F., Cordero Ramajo J., Liarte Trías I., Benítez Segura A., Bajén Lázaro M., Ferrán Sureda N., Puchal Añé R., Gámez Cenzano C., Influence of radioactive concentration and storage time on radiochemical purity of 18F-FDG. Rev Esp Med Nucl. (2006) 45:20-5; Hjelstuen O.K., Svadberg A., Olberg D.E., Rosser M., Standardization of fluorine-18 manufacturing processes: new scientific challenges for PET. Eur J Pharm Biopharm. (2011) 78:307-13).

### Problem to be solved

While the aforementioned state of the art studies only give solutions on enhancing the stability of the final radiolabeled product, it is worthwhile to tackle the radiolysis upstream, mainly during the labelling step. It is of primary importance of maximizing the [¹⁸F]-fluoride incorporation during the radiolabeling step to significantly increase the production yield, the number of injectable doses per production, and consequently to reduce the cost of doses to be administered.

Moreover, as PET imaging relies on the detection of positrons emitted by the decay of [¹⁸F]-labeled radiotracers, a higher incorporation of [¹⁸F] in the precursor molecule results in a higher specific activity of the radiotracer. Radiotracers with high specific activity provide better sensitivity and accuracy in detecting the target biological process or molecule within the body. Moreover, higher incorporation allows for the synthesis of radiotracers with a lower mass of the cold (non-radioactive) component. This is important because even a small mass of the carrier molecule can interfere with the biological processes under investigation, potentially leading to biased images or unwanted side effects. Finally, radiotracers with high specific activity provide sharper PET images. This improved resolution allows for better localization and quantification of the biological target, contributing in accurate diagnosis and treatment monitoring.

Maximizing the incorporation of [¹⁸F] in a precursor molecule is thus crucial for producing high-quality radiotracers with optimal sensitivity, accuracy, and imaging resolution, but also for a more efficient patient outcome as it will result in an enhancement of the radiotracer production outcome. However, achieving high incorporation rates, particularly at high activity levels, is challenging due to the increased likelihood of radiolysis (where high-energy radiation breaks chemical bonds in the precursor molecule), reaction complexity, and shorter reaction times. Overcoming these challenges requires careful optimization of reaction conditions.

### Aims of the Invention

The present invention aims at avoiding decomposition reactions, i.e. radiolysis resulting from high radioactivity concentrations, of radiosynthetic intermediates used in the synthesis of [¹⁸F]-labeled radiotracers by the use of radical scavengers during the nucleophilic [¹⁸F]-fluorination step. In other words, the invention aims at stabilizing the radiochemical yields of radiochemical synthesis of [¹⁸F]-labeled radiotracers obtained through nucleophilic aliphatic or aromatic substitution whatever the level of starting radioactivity used.

### Disclosure of the Invention

The present invention relates to the stabilization toward radiolysis of radiosynthetic intermediates, including cold intermediates such as the precursor to be radiolabelled, by the use of radical scavengers during the nucleophilic [¹⁸F]-fluorination step of a radiochemical synthesis of a [¹⁸F]-labeled radiotracer.

Depending on the precursor to be labeled, the nucleophilic [¹⁸F]-fluorination step can be classified as aliphatic or aromatic. The former is encountered in the [¹⁸F]-FDG synthesis. The usual synthesis of [¹⁸F]-FDG is a two-step process consisting of two chemical reactions: a nucleophilic [¹⁸F]-fluorination of a mannose triflate precursor (1,3,4,6-tetra-O-acetyl-2-O-trifluoromethanesulfonyl-β-D-mannopyranose) followed by a hydrolysis step of some protecting groups. The substitution reaction is accomplished in polar aprotic solvents, such as acetonitrile, after a few drying steps. At high activity, the substitution reaction can lead to numerous by-products, due to radiolysis, negatively impacting the production yield (**examples 1-12**). The invention aims to stabilizing the [¹⁸F]-labeled radiotracer production outcome by stabilizing the labeling yield whatever the starting activity, through the addition of a radical scavenger in the reaction vessel.

Manufacturing of a [¹⁸F]-labelled molecule through a nucleophilic [¹⁸F]-fluorination step begins with the production of [¹⁸F]fluoride in a cyclotron. Two important characteristics of cyclotrons pertaining to the production of [¹⁸F]-fluoride are the proton beam energy and the beam current being used. These two factors, along with the target volume, will determine how much [¹⁸F]-fluoride quantity, i.e. the activity, can be produced in a given amount of time. To some extent, the higher the energy and the beam current, the greater the fluoride production. All of the nowadays commercially available cyclotrons are capable of generating a beam with a current of at least 50 µA. The liquid targets used for [¹⁸F] production are usually not run above 100 µA because of the limitation imposed by the dissipation of heat generated during irradiation. Typically, a given target with a given operating pressure has an optimum current, at which the production rate is the highest. This need not be the highest possible current. Most powerful and efficient today cyclotrons can produce up to 1480 GBq of [¹⁸F]-fluoride. Knowing that radiolysis can already occur at activities as low as ~3,5 GBq, it is crucial to find solutions against radiolysis at such activity levels to stabilize the labeling yield and thus the [¹⁸F]-labeled radiotracer production outcome whatever the starting activity.

In an effort to stabilize the aforementioned yields, the inventors have surprisingly found that the radical scavenger species could be a polar protic compound, if added in specific concentrations (**examples 13-28**).

In some embodiments, the radical scavenger is a primary alcohol or alkanol. In some preferred embodiments, the radical scavenger is ethanol.

In some embodiments, said [18F]-labelled radiotracer are selected from, but not limited to, [¹⁸F]-NaF, [¹⁸F]-FDG, [¹⁸F]-FMISO, [¹⁸F]-FLT, [¹⁸F]-FAZA, [¹⁸F]-FCH, [¹⁸F]-FDOPA, [¹⁸F]-FES, [¹⁸F]-FET, [¹⁸F]-FAPI derivatives.

In some embodiments, the radical scavenger concentration range is from 5000 ppm to 40000 ppm.

In some preferred embodiments, the radical scavenger concentration range is from 10000 ppm to 30000 ppm.

In some another preferred embodiments, the radical scavenger concentration range is from 15000 ppm to 25000 ppm.

The synthesis of [¹⁸F]-labelled molecules using the method according to the present disclosure may be carried out manually, or using an automated radiosynthesizer. Homemade or commercially available automated radiosynthesizers are designed for use in an appropriate hot cell that provides suitable radiation shielding to protect the operator from potential radiation dose. When using a cassette, the automated radiosynthesizer can produce a variety of different radiopharmaceuticals with minimal risk of cross-contamination by simply changing the cassette. This approach also has the advantage of simplifying setup, thereby reducing the risk of operator error, allowing the cassette to be changed quickly between runs, and improving compliance with GMP regulations, which is the purpose of the present invention.

The term "cassette" means an equipment unit constructed so that the entire unit fits removably and interchangeably on an automatic synthesizer device (as defined above) in such a way that the mechanical movement of moving parts of the synthesizer controls the operation of the cassette from outside the cassette. Suitable cassettes consist of a linear array of valves, each connected to a port to which reagents or vials can be connected, either by needle insertion into a vial sealed with an inverted septum or by gastight connectors. The cassette is versatile as it usually has several positions where reagents can be attached, as well as several positions suitable for attaching syringes or chromatography cartridges (e.g. for solid phase extraction or SPE). The cassette always includes at least one reaction vessel.

This invention is not dependent on the details of the above examples and should apply to any process that uses a nucleophilic [¹⁸F]-fluorination step.

### EXAMPLES

### Examples 1-12: synthesis of [¹⁸F]-FDG at high activity without the presence of a radical scavenger during the labeling step

### General procedure

The automated synthesis of [¹⁸F]-FDG was performed on the AllinOne^{®} synthesizer (Trasis SA, Belgium). Briefly, [¹⁸F]-fluoride obtained from the bombardment of enriched [¹⁸O]-water was trapped on a carbonated QMA Plus light cartridge (130 mg of sorbent, the QMA was used as received). An aliquot of the K₂CO₃/K₂₂₂ containing eluent (300 µL) was used to elute the trapped [¹⁸F]-fluoride to the reaction vessel. After evaporation of the water by azeotropic drying, an aliquot of the precursor was added, to the reactor and the labelling was carried out at 115 °C during 1.5 min. The intermediate, [¹⁸F]-FTAG was subsequently trapped and deprotected on the tC18 cartridge, by the mean of passing an aliquot of a sodium hydroxide solution trough the cartridge. An aliquot of water was used to elute crude [¹⁸F]FDG resulting from the reaction, which was subsequently neutralized with an aliquot of citrate buffer solution. [¹⁸F]FDG was eventually purified through two purification cartridges and sent to the final product vial. To be commercially viable, Non-Decay Corrected (NDC) yield of minimum 65% in [¹⁸F]-FDG is mandatory.

### Results

| **Ex. #** | **Run I.D.** | **Starting ¹⁸F activity (GBq)** | **EtOH content (ppm)** | **Non-decaycorrected yield (%)** |
|---|---|---|---|---|
| **1** | FDGQ-230626-A1-RGE | 89,7 | 0 | 74,2 |
| **2** | FDGD-230629-A1-SCE | 101,5 | 0 | 77,1 |
| **3** | FDGD-230629-A2-SCE | 105,0 | 0 | 74,1 |
| **4** | FDGD-230714-A2-SCE | 248,8 | 0 | 73,7 |
| **5** | FDGD-230714-A1-SCE | 355,2 | 0 | 72,4 |
| **6** | FDGQ-230807-A1-SCE | 383,5 | 0 | 70,3 |
| **7** | FDGQ-230724-A1-SCE | 452,3 | 0 | 72,9 |
| **8** | FDGQ-230710-A1-SCE | 621,6 | 0 | 60,0 |
| **9** | FDGD-230720-A1-SCE | 634,0 | 0 | 57,6 |
| **10** | FDGQ-230727-A1-SCE | 680,3 | 0 | 68,6 |
| **11** | FDGD-230703-A1-SCE | 725,9 | 0 | 58,6 |
| **12** | FDGQ-230608-A1-RGE | 795,0 | 0 | 60,3 |

### Conclusions

Results show that the yield decreases almost linearly with the starting activity, up to 600 GBq. Beyond this point, the yield drops significantly and, in any case, is not stable at all. All these variations are due to radiolytic effects that reduce the labeling yield. These examples demonstrate a critical need to stabilize fluorination yields, regardless of the starting activity.

### Examples 13-24: Synthesis of [¹⁸F]-FDG in the presence of ethanol during the labeling step at low activity

### General procedure

Illustrative examples of the present invention is the automated synthesis of [¹⁸F]-FDG on the AllinOne^{®} synthesizer (Trasis SA, Belgium). Briefly, [¹⁸F]-fluoride obtained from the bombardment of enriched [¹⁸O]-water was trapped on a carbonated QMA Plus light cartridge (46 mg of sorbent, the QMA was used as received). An aliquot of the K₂CO₃/K₂₂₂ containing eluent (300 µL) was used to elute the trapped [¹⁸F]-fluoride to the reaction vessel. After evaporation of the water by azeotropic drying, an aliquot of the precursor was added, together with varying quantities of ethanol, to the reactor and the labelling was carried out at 115 °C during 1.5 min. The intermediate, [¹⁸F]-FTAG was subsequently trapped and deprotected on the tC18 cartridge, by the mean of passing an aliquot of a sodium hydroxide solution trough the cartridge. An aliquot of water was used to elute crude [¹⁸F]FDG resulting from the reaction, which was subsequently neutralized with an aliquot of citrate buffer solution. [¹⁸F]FDG was eventually purified through two purification cartridges and sent to the final product vial. To be commercially viable, Non-Decay Corrected (NDC) yield of minimum 65% in [¹⁸F]-FDG is mandatory.

### Results

| **Ex. #** | **Run I.D.** | **Starting ¹⁸F activity (MBq)** | **EtOH content (ppm)** | **Non-decaycorrected yield (%)** |
|---|---|---|---|---|
| **13** | FDGD-230913-A1-SBN | 240 | 5000 | 72 |
| **14** | FDGD-230913-A2-SBN | 196 | 5000 | 71 |
| **15** | FDGD-230913-B1-SBN | 165 | 10000 | 69 |
| **16** | FDGD-230913-B2-SBN | 200 | 10000 | 65 |
| **17** | FDGD-230914-A1-SBN | 528 | 20000 | 72 |
| **18** | FDGD-230914-A2-SBN | 489 | 20000 | 69 |
| **19** | FDGQ-230928-A1-ACC | 181 | 20000 | 73 |
| **20** | FDGQ-230928-A2-ACC | 180 | 20000 | 67 |
| **21** | FDGQ-230928-A3-ACC | 173 | 20000 | 70 |
| **22** | FDGQ-230928-A4-ACC | 211 | 20000 | 69 |
| **23** | FDGD-230919-B1-SBN | 161 | 30000 | 16 |
| **24** | FDGD-230919-A1-SBN | 227 | 40000 | 36 |

### Conclusion

These examples prove that it is possible to perform a nucleophilic [¹⁸F]-fluorination in the presence of a polar protic solvent, e.g. ethanol, at the condition that its concentration remains under 30000 ppm. This result was unexpected and is innovative compared to the state of the art. Under 30000 ppm, the [¹⁸F]-fluoride anions remain fully reactive.

### Examples 25-27: Synthesis of [18F]-FDG in the presence of ethanol during the labeling step at high activity

### General procedure

The automated synthesis of [¹⁸F]-FDG was performed on the AllinOne^{®} synthesizer (Trasis SA, Belgium). Briefly, [¹⁸F]-fluoride obtained from the bombardment of enriched [¹⁸O]-water was trapped on a carbonated QMA Plus light cartridge (46 mg of sorbent, the QMA was used as received). An aliquot of the K₂CO₃/K₂₂₂ containing eluent (300 µL) was used to elute the trapped [¹⁸F]-fluoride to the reaction vessel. After evaporation of the water by azeotropic drying, an aliquot of the precursor was added, together with varying quantities of ethanol, to the reactor and the labelling was carried out at 115 °C during 1.5 min. The intermediate, [¹⁸F]-FTAG was subsequently trapped and deprotected on the tC18 cartridge, by the mean of passing an aliquot of a sodium hydroxide solution trough the cartridge. An aliquot of water was used to elute crude [¹⁸F]FDG resulting from the reaction, which was subsequently neutralized with an aliquot of citrate buffer solution. [¹⁸F]FDG was eventually purified through two purification cartridges and sent to the final product vial. To be commercially viable, Non-Decay Corrected (NDC) yield of minimum 65% in [¹⁸F]-FDG is mandatory.

### Results

| **Ex. #** | **Run I.D.** | **Starting ¹⁸F activity (GBq)** | **EtOH content (ppm)** | **Non-decay-corrected yield (%)** |
|---|---|---|---|---|
| **25** | FDGD-231009-A1 | 684,4 | 20000 | 69,0 |
| **26** | FDGD-231009-A2 | 737,0 | 20000 | 65,4 |
| **27** | FDGD-231019-A1 | 822,9 | 20000 | 65,3 |

### Conclusion

These examples prove that the ethanol can be used to stabilize the nucleophilic [¹⁸F]-fluorination yields at high activity. At such starting activities, a production yield which is stabilized and increased by 10 percentage points translates into tens of additional patient doses, for each production.

## Claims

1. A method to stabilize the production yield of [¹⁸F]-labelled radiotracers by the addition of a radical scavenger in the reaction media of the nucleophilic [¹⁸F]-fluorination step.

2. The method of claim 1 wherein said [18F]-labelled radiotracer is selected from the group consisting of [¹⁸F]-NaF, [¹⁸F]-FDG, [¹⁸F]-FMISO, [¹⁸F]-FLT, [¹⁸F]-FAZA, [¹⁸F]-FCH, [¹⁸F]-FDOPA, [¹⁸F]-FES, [¹⁸F]-FET, and [¹⁸F]-FAPI derivatives.

3. The method of claim 1 wherein the radical scavenger is a polar protic solvent.

4. The method of claim 1 wherein the radical scavenger is a primary alkanol.

5. The method of claim 1 wherein the radical scavenger is methanol or ethanol.

6. The method of claim 1 wherein the radical scavenger is ethanol.

7. The method of claim 1 wherein the radical scavenger concentration range is from 5000 to 40000 ppm.

8. The method of claim 1 wherein the radical scavenger concentration range is from 10000 to 30000 ppm.

9. The method of claim 1 wherein the radical scavenger concentration range is from 15000 to 25000 ppm.

10. The method of claim 1 wherein the [¹⁸F]-labelled radiotracer is [¹⁸F]-FDG, the radical scavenger is ethanol and the concentration range of the radical scavenger is from 15000 to 25000 ppm.
